Europäisches Patentamt
European Patent Office
Office européen des brevets

(19) (11) Publication number: **0 037 851 A2**

(12) **EUROPEAN PATENT APPLICATION**

(21) Application number: **80105947.8**

(22) Date of filing: **01.10.80**

(51) Int. Cl.³: **A 01 N 43/40**, A 01 N 53/00, C 07 D 213/64, C 07 D 407/12

(30) Priority: **15.04.80 JP 49764/80**

(43) Date of publication of application: **21.10.81**
**Bulletin 81/42**

(84) Designated Contracting States: **CH DE FR GB IT LI NL**

(71) Applicant: **SUMITOMO CHEMICAL COMPANY, LIMITED, 15 Kitahama 5-chome Higashi-ku, Osaka-shi Osaka-fu (JP)**

(72) Inventor: **Itaya, Nobushige, 5-3-101, Ryodocho, Nishinomiya-shi (JP)**
Inventor: **Hirano, Masachika, 6-H-104, Tenno-2-chome, Ibaraki-shi (JP)**
Inventor: **Matsuo, Noritada, 29-2, Aza Yamamichi, Minamino, Itami-shi (JP)**

(74) Representative: **Vossius-Vossius-Tauchner-Heunemann-Rauh Patentanwälte, P.O.Box 860767 Siebertstrasse 4, D-8000 München 86 (DE)**

(54) **Carboxylates, a process for their production, an insecticidal and/or acaricidal composition and the use of the compounds as insecticides and/or acaricides.**

(57) The invention relates to carboxylates of the formula (I),

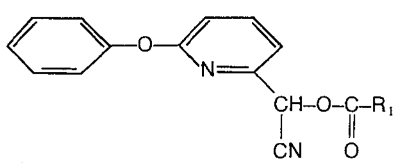

wherein $R_1$ is

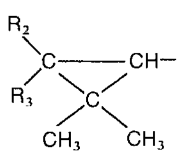 (II) or 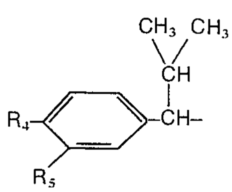 (III)

wherein $R_2$ is a hydrogen atom or a methyl group; $R_3$ is a 2-methyl-1-propenyl group when $R_2$ is a hydrogen atom, and $R_3$ is a methyl group when $R_2$ is a methyl group; each of $R_4$ and $R_5$ is a hydrogen atom, a fluorine, chlorine or bromine atom, or a methyl, methoxy or 3,4-methylenedioxy group, with the proviso that $R_4$ and $R_5$ are not hydrogen atoms at the same time, a process for their production, insecticidal and/or acaricidal compositions containing these compounds as active ingredients, and the use of the compounds as insecticides and/or acaricides.

EP 0 037 851 A2

0037851

"Carboxylates, a process for their production, an insecticidal and/or acaricidal composition and the use of the compounds as insecticides and/or acaricides"

Priority: April 15, 1980, Japan, No. 49 764/80

This invention relates to carboxylic acid esters , a process for preparing such esters, an insecticidal and/or acaricidal composition containing said esters as active ingredient and the use of the compounds as insecticides and/or acaricides. The esters have the general formula (I):

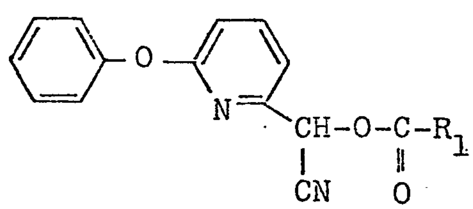

(I)

wherein $R_1$ is

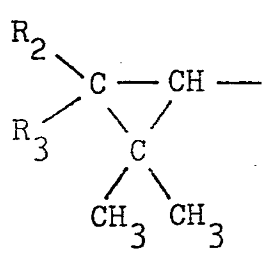

(II) or

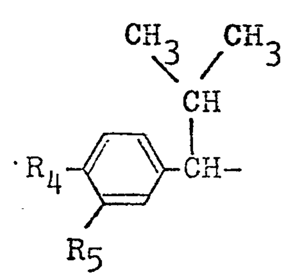

(III)

wherein $R_2$ is a hydrogen atom or a methyl group; $R_3$ is a 2-methyl-1-propenyl when $R_2$ is a hydrogen atom, and $R_3$ is a methyl group when $R_2$ is a methyl group;

each of $R_4$ and $R_5$ is a hydrogen atom, a fluorine, chlorine or bromine atom, or a methyl, methoxy or methylenedioxy group, with the proviso that when $R_4$ and $R_5$ are not hydrogen atoms at the same time.

Various kinds of cyclopropanecarboxylic acid ester type insecticides are known, and some of such insecticides are found in the list of the pyrethrum compositions. Among many currently used insecticides, those of the pyrethrum compositions are widely used for controlling the sanitary harmful insects and the harmful insects in agriculture and horticulture because of their many advantageous properties such as low toxicity to man and beast, rapid effect on the harmful insects and unlikeliness of making the harmful insects resistant to them in addition to their inherent insecticidal activity. These pyrethrum insecticides, however, have drawbacks that they are expensive and subject to certain restrictions in the scope of their applications, and hence a plurality of analogous compounds have been synthesized by many scientists.

As a result of the studies in search for a compound which possesses all of said merits of the pyrethroid type insecticides and which is still acceptable in the economical sense, the present inventors found that the compound of this invention represented by the general formula (I) has the properties which meet these requirements.

The compound of this invention proves effective

particularly against the following harmful insects:

1. Hemiptera
   1. Delphacidae such as Sogatella furcifera, Nilaparvata lugens, Laodelphax striatellus
   2. Deltocephalidae such as Nephotettix cincticeps, Tettigella viridis, Inazuma dorsalis
   3. Aphididae such as Rhopalosiphum padi, Myzus persicae
   4. Pentatomidae such as Nezara antennata, Eysarcaris ventralis
2. Lepidoptera such as Archips fumiferana, Chilo suppressalis, Cuapha locrocis medinalis, Galleria mellonella, Dendrolimus spectabilis, Malacosoma neustria, Spodoptera litura, plutella xylostella
3. Coleoptera such as Tribolium castaneum, Oulema oryzae, Echinocnemus squameus
4. Diptera such as Musca domestica, Aedes aegypti, Anopheles sp, Culex pipiens, Agromyza oryzae
5. Orthoptera such as Blattella germanica, Oxya yezoensis
6. Acarina such as Tetranychus cinnabarinus, Tetranychus urticae, Oligonychus hondoensis, Panonychus citri

The compound of this invention represented by the general formula (I) can be obtained in a high yield according to a process which comprises reacting a carboxylic acid of the general formula (IV):

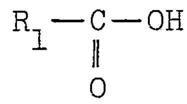

(IV)

wherein $R_1$ is as defined above, or a reactive derivative thereof with an alcohol or its reactive derivative of the general formula (V):

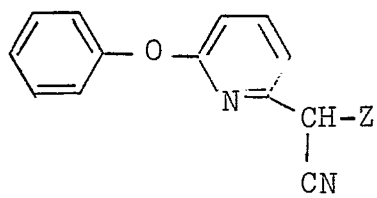

(V)

wherein Z is a hydroxy group or a chlorine or bromine atom, or a process which comprises reacting a carboxylic acid halide of the general formula (VI):

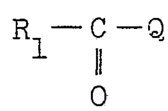

(VI)

wherein Q is a chlorine or bromine atom, and $R_1$ is as defined above, with an aldehyde of the formula (VII):

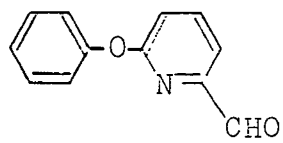

(VII)

and an alkali metal cyanide.

The "reactive derivative" of a carboxylic acid represented by the general formula (IV) as used in the above-said reaction may be, for example, a carboxylic acid halide, a carboxylic anhydride, a salt of a tertiary organic base of a carboxylic acid or an alkali metal salt of a carboxylic acid.

The compounds according to this invention include the optical isomers associated with the asymmetric carbon atom in the alcohol and acid moieties, and all of such isomers are also included within the scope of this invention.

Some ways for preparing the carboxylic acid esters according to this invention are shown below.

<u>Synthesis process A</u> (process comprising a reaction of an alcohol and a carboxylic acid halide)

An alcohol of the formula (VIII):

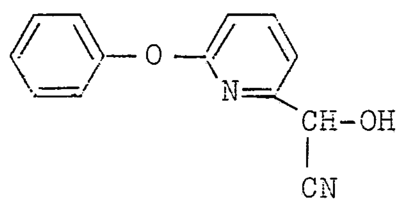

(VIII)

and a carboxylic acid halide of the general formula (VI):

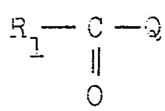

(VI)

wherein $R_1$ and Q are as defined above, preferably an acid chloride, are reacted in an inert solvent (such as benzene, toluene, ether, hexane, etc.) in the presence of an acid-binding agent (such as pyridine, triethylamine, etc.) at an internal temperature of -30° to 100°C for a period of 30 minutes to 10 hours to obtain the objective ester.

<u>Synthesis process B</u> (process comprising a reaction of an alcohol and a carboxylic acid)

An alcohol of the formula (VIII):

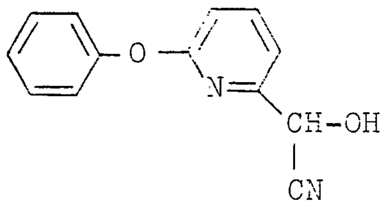

(VIII)

and a carboxylic acid of the general formula (IV):

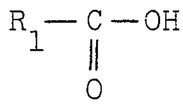

(IV)

wherein $R_1$ is as defined above, are reacted in an inert solvent (such as benzene, toluene, xylene, etc.) in the presence of a dehydrating-condensing agent (such as dicyclohexylcarbodiimide, etc.) at an internal temperature of 0° to 150°C for a period of 30 minutes to 10 hours to obtain the objective ester.

<u>Synthesis process C</u> (process comprising a reaction of a halide and a salt of a tertiary organic base of a carboxylic acid)

A halide of the general formula (IX):

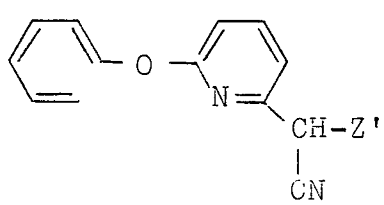

(IX)

wherein Z' is chlorine or bromine atom, and a carboxylic acid of the general formula (IV):

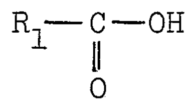 (IV)

wherein $R_1$ is as defined above, are reacted in an inert solvent (such as acetone, benzene, dioxane, etc.) in the presence of a tertiary organic base (such as triethylamine, trimethylamine, etc.) at an internal temperature of 0° to 150°C for a period of 30 minutes to 10 hours to obtain the objective ester.

<u>Synthesis process D</u> (process comprising a reaction of a halide and an alkali metal salt of a carboxylic acid)

A halide of the general formula (IX):

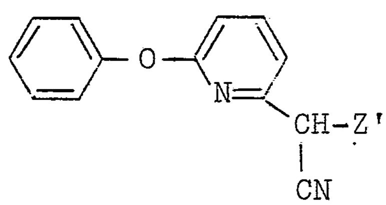 (IX)

wherein Z' is chlorine or bromine atom, and an alkali metal salt of a carboxylic acid of the general formula (X):

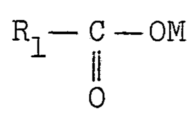 (X)

wherein M is an alkali metal and $R_1$ is as defined above, are reacted in a water-inert solvent (such as toluene, heptane, benzene, etc.) two-phase system in the presence of a phase transfer catalyst (such as tetra-n-butylammonium bromide, benzyltriethylammonium chloride, etc.) at an internal temperature of 0° to 150°C for a period of 30 minutes to 10 hours to obtain the objective ester.

Synthesis process E (process comprising a reaction of an aldehyde, an alkali metal cyanide and an acid halide)

E-1

An aldehyde of the formula (VII)

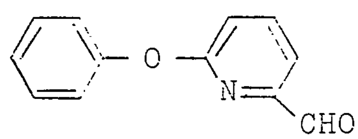

(VII)

as an alkali metal cyanide and a carboxylic acid halide of the general formula (VI):

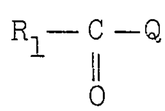

(VI)

wherein Q and $R_1$ are as defined above, are reacted in an inert solvent (such as benzene, toluene, etc.) in the presence of a phase transfer catalyst (such as dibenzo-18-crown-6, dicyclohexyl-18-crown-6, etc.) at an internal temperature of 0° to 150°C for a period of

30 minutes to 20 hours to obtain the objective ester.

E-2

An aldehyde of the formula (VII):

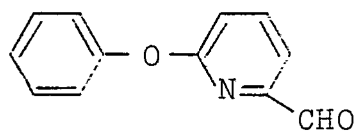

(VII)

an alkali metal cyanide and a carboxylic acid halide of the general formula (VI):

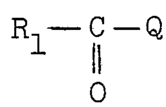

(VI)

wherein Q and $R_1$ are as defined above, are reacted in a water-inert solvent (such as benzene, hexane, toluene, etc.) two-phase system in the presence of a phase transfer catalyst (such as tetra-n-butylammonium bromide, benzyltriethylammonium chloride, etc.) at an internal temperature of 0° to 100°C for a period of 30 minutes to 10 hours to obtain the objective ester.

Listed below are the carboxylic acid esters of this invention which were synthesized according to the above-described synthesis processes.

| Compound No. | Structural formula and chemical name | Refractive index or properties | Elementary analysis |
|---|---|---|---|
| (1) | 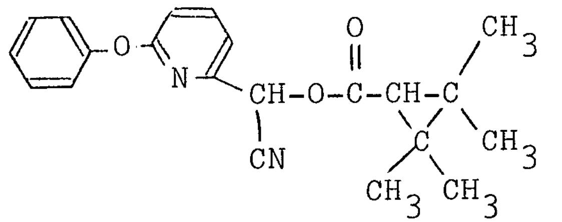<br>6-phenoxy-2-pyridyl-α-cyanomethyl 2,2,3,3-tetramethylcyclopropane-carboxylate | $n_D^{20}$ 1.5486 | C(%) H(%) N(%)<br>Calcd. for $C_{21}H_{22}N_2O_3$:<br>71.98 6.33 7.99<br>Found: 72.33 6.21 7.61 |
| (2) | 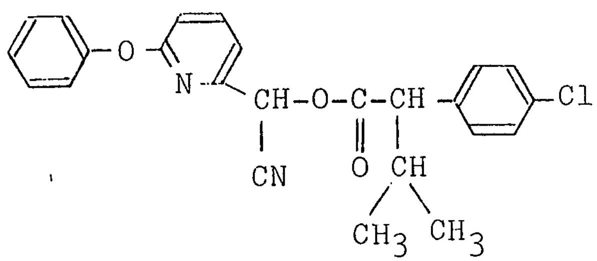<br>6-phenoxy-2-pyridyl-α-cyanomethyl 2-(4-chlorophenyl)isovalerate | $n_D^{20}$ 1.5651 | C(%) H(%) N(%)<br>Calcd. for $C_{24}H_{21}N_2O_3Cl$:<br>68.48 5.03 6.66<br>Found: 68.71 5.22 6.45 |

- Cont'd -

| | | | |
|---|---|---|---|
| (3) | 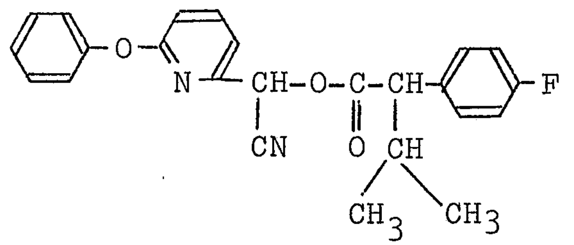 6-phenoxy-2-pyridyl-α-cyanomethyl 2-(4-fluorophenyl)isovalerate | $n_D^{24}$ 1.5483 | C(%) H(%) N(%)<br>Calcd. for $C_{24}H_{21}N_2O_3F$:<br>71.27 5.23 6.93<br>Found: 71.21 5.00 6.82 |
| (4) | 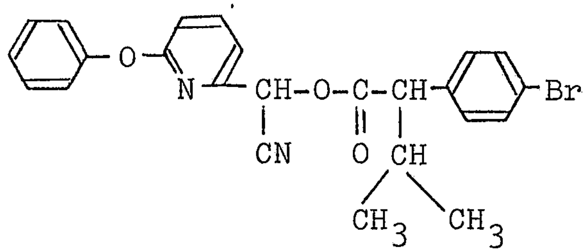 6-phenoxy-2-pyridyl-α-cyanomethyl 2-(4-bromophenyl)isovalerate | $n_D^{26}$ 1.5732 | C(%) H(%) N(%)<br>Calcd. for $C_{24}H_{21}N_2O_3Br$:<br>61.94 4.55 6.02<br>Found: 62.09 4.76 6.18 |

- Cont'd -

| | | | |
|---|---|---|---|
| (5) | 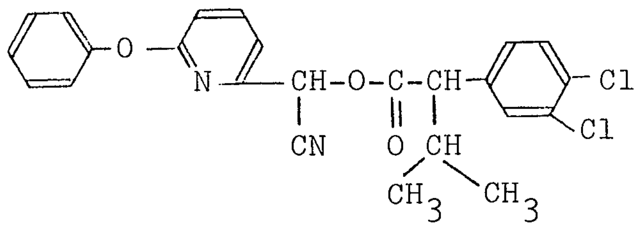<br>6-phenoxy-2-pyridyl-α-cyanomethyl 2-(3,4-dichlorophenyl)isovalerate | $n_D^{26.5}$ 1.5715 | C(%) H(%) N(%)<br>Calcd. for $C_{24}H_{20}N_2O_3Cl_2$:<br>63.30 3.51 6.15<br>Found: 63.12 3.48 6.29 |
| (6) | 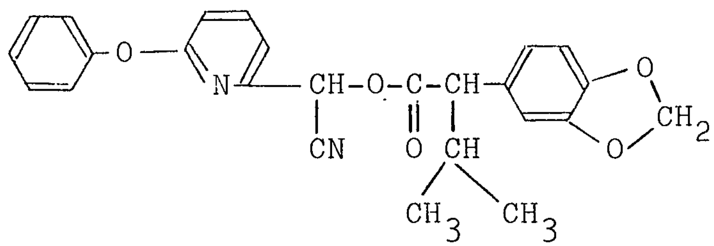<br>6-phenoxy-2-pyridyl-α-cyanomethyl 2-(3,4-methylenedioxy)isovalerate | $n_D^{23.5}$ 1.5746 | C(%) H(%) N(%)<br>Calcd. for $C_{25}H_{22}N_2O_5$:<br>69.75 5.15 6.51<br>Found: 69.72 5.04 6.85 |

- Cont'd -

| | | | |
|---|---|---|---|
| (7) | 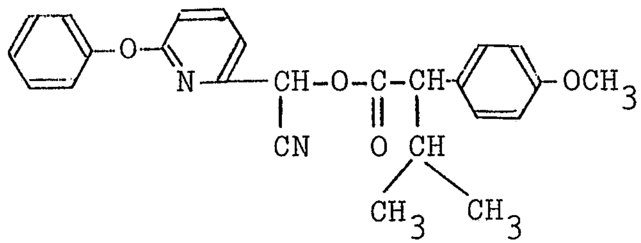 6-phenoxy-2-pyridyl-α-cyanomethyl 2-(4-methoxyphenyl)isovalerate | $n_D^{28}$ 1.5594 | C(%) H(%) N(%)<br>Calcd. for $C_{25}H_{24}N_2O_4$:<br>72.10 5.81 6.73<br>Found: 72.42 5.44 6.71 |
| (8) | 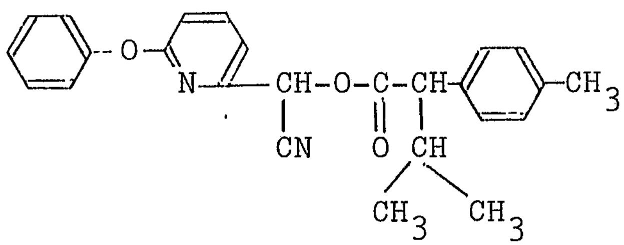 6-phenoxy-2-pyridyl-α-cyanomethyl 2-(4-methylphenyl)isovalerate | $n_D^{25.5}$ 1.5539 | C(%) H(%) N(%)<br>Calcd. for $C_{25}H_{24}N_2O_3$:<br>74.98 6.04 7.00<br>Found: 75.11 6.21 7.35 |

- Cont'd -

| | | | |
|---|---|---|---|
| (9) | 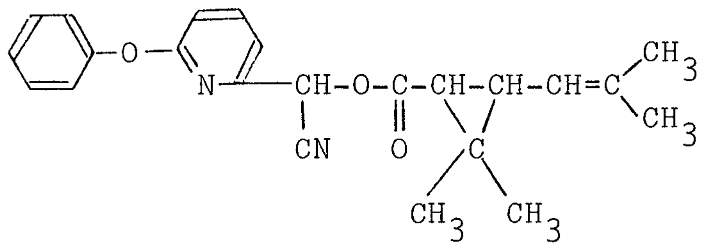<br>6-phenoxy-2-pyridyl-α-cyanomethyl d,l-cis,trans-chrysanthemate | $n_D^{17}$ 1.5487 | C(%) H(%) N(%)<br>Calcd. for $C_{23}H_{24}N_2O_3$:<br>73.38 6.43 7.44<br>Found: 73.20 6.35 7.21 |

The syntheses of the compounds according to this invention are described in more detail hereinbelow by way of the examples.

Example 1 Synthesis of Compound (2)

To a solution of 1.36 g (6.0 mmoles) of 6-phenoxy-2-pyridyl-α-cyanomethanol in 10 ml of anhydrous benzene, was added 0.95 g (12.0 mmoles) of pyridine. To the resulting mixture, with stiring and maintaining a temperature below 5°C. in an ice bath, was added dropwise a solution of 1.39 g (6.0 mmoles) of 2-(4-chlorophenyl)isovaleric acid chloride in 5 ml of anhydrous benzene. After the addition was completed, the reaction mixture was allowed to react at room temperature and stirred overnight. After dissolution of the precipitated pyridine hydrochloride by addition of water to the reaction mixture, the aqueous layer was separated. The organic layer was washed with 5% hydrochloric acid, saturated aqueous sodium hydrogencarbonate solution and then saturated aqueous sodium chloride solution. After drying over anhydrous sodium sulfate, the organic layer was concentrated under reduced pressure. The residue was chromatographed on silica gel to give 2.37 g of 6-phenoxy-2-pyridyl-α-cyanomethyl 2-(4-chlorophenyl)-isovalerate as a pale yellow liquid (yield 94%).

Example 2 Synthesis of Compound (9)

1.73 Grams (6.0 mmol) of 6-phenoxy-2-pyridyl-

α-cyanomethyl bromide and 1.21 g (7.2 mmol) of dl-cis,trans-chrysanthemic acid were dissolved in 20 ml of acetone, and to this solution was added dropwise a solution formed by dissolving 0.81 g (8.0 mmol) of triethylamine in 5 ml of acetone, under stirring at 15°-20°C. After completion of said dropwise addition, the mixed solution was refluxed under heating for 2 hours and then cooled naturally. The triethylamine bromate separated from the resulting reaction solution was filtered and concentrated, and the residue was purified by silica gel-packed column chromatography to obtain 2.06 g of 6-phenoxy-2-pyridyl-α-cyanomethyl dl-cis,trans-chrysanthemate in the form of a pale yellow liquid. (Yield: 91%).

Example 3 Synthesis of Compound (7)

0.37 Gram (7.5 mmol) of sodium cyanide and 0.25 g (1.1 mmol) of benzyltriethylammonium chloride were dissolved in 5 ml of water, and to this solution was added dropwise, under stirring at room temperature, a solution formed by dissolving 1.00 g (5.0 mmol) of 6-phenoxy-2-pyridylcarboaldehyde and 1.19 g (5.25 mmol) of 2-(4-methoxyphenyl)-isovaleric acid chloride in 10 ml of toluene, and after completion of this addition, the mixed solution was stirred at the same temperature for 5 hours. The resulting reaction solution was washed with an aqueous saturated sodium

chloride solution and then dried with anhydrous sodium sulfate. When the solvent was distilled off, there was obtained 1.96 g of 6-phenoxy-2-pyridyl-α-cyanomethyl 2-(4-methoxyphenyl)-isovalerate as a pale yellow liquid. (Yield: 94%).

Example 4 Synthesis of Compound (1)

0.44 Gram (9.0 mmol) of sodium cyanide and 0.1 g of dibenzo-18-crown-6 were suspended in 10 ml of benzene, and to this suspension was added dropwise under stirring at room temperature a solution formed by dissolving 1.20 g (6.0 mmol) of 6-phenoxy-2-pyridylcarbo-aldehyde and 1.01 g (6.3 mmol) of 2,2,3,3-tetramethyl-cyclopropanecarboxylic acid chloride in 10 ml of benzene. After completion of said dropwise addition, the mixed solution was further stirred overnight and the resulting reaction solution was washed with an aqueous sodium chloride solution and then concentrated. The residue was purified by silica gel-packed column chromatography to obtain 2.00 g of 6-phenoxy-2-pyridyl-α-cyanomethyl 2,2,3,3-tetramethylcyclopropanecarboxylate in the form of a pale yellow liquid. (Yield: 95%).

Example 5 Synthesis of Compound (4)

To a solution of 1.73 g (6.0 mmol) of 6-phenoxy-2-pyridyl-α-cyanomethyl bromide in 10 ml of toluene was added a solution formed by dissolving 2.01 g (7.2 mmol)

of sodium 2-(4-bromophenyl)-isovalerate and 0.081 g (0.25 mmol) of tetra-n-butylammonium bromide in 10 ml of water, and the mixed solution was stirred at an internal temperature of 70° - 80°C for 5 hours. The resulting reaction solution was washed with an aqueous sodium chloride solution and then dried with anhydrous sodium sulfate. Upon distilling off the solvent, there was obtained 2.65 g of 6-phenoxy-2-pyridyl-α-cyanomethyl 2-(4-bromophenyl)-isovalerate in the form of an orange liquid. (Yield: 95%).

Example 6 Synthesis of Compound (6)

To a suspension of 0.44 g (9.0 mmoles) of sodium cyanide and 0.10 g of dibenzo-18-crown-6 in 10 ml of anhydrous benzene, was added dropwise, with stirring at room temperature, a solution of 1.20 g (6.0 mmoles) of 6-phenoxy-2-pyridylcarboaldehyde and 1.52 g (6.30 mmoles) of 2-(3,4-methylenedioxyphenyl)-isovaleric acid chloride in 10 ml of anhydrous benzene. Thereafter, the mixture was further stirred overnight. The reaction mixture was washed with saturated aqueous sodium chloride solution and the solvent was evaporated. The residue was chromatographed on silica gel to give 2.40 g of 6-phenoxy-2-pyridyl-α-cyanomethyl 2-(3,4-methylenedioxyphenyl)-isovalerate as a pale yellow

liquid (yield 93%).

The compounds of this invention obtained according to the above-described processes may be put to use alone with no addition of other components, but in order to facilitate the use as a controlling composition, the compound is usually blended with a carrier and prepared into a formulation convenient for use, and such preparation, if necessary, may be further diluted in actual use.

Preparation of the compounds of this invention into a formulation for use can be attained without requiring any specific condition but according to the ordinary agricultural chemicals formulation standards, and the compounds may be prepared into any desired form such as emulsifiable concentrate, wettable powder, dust, granules, fine granules, oil solution, aerosol, heating fumigant (mosquito coil, electric mosquito killer, etc.), spray such as fogging agent, non-heating fumigant, toxic bait, etc., according to the methods well known to those skilled in the art, and these preparations may be put to actual use in various ways to suit the purpose of use.

Further, these compounds may be used in admixture of two or more of them to effect more excellent insecticidal effect. It is also possible to mix with the compounds the pyrethroid synergists such as α-(2-(2-butoxyethoxy)ethoxy)-4,5-methylenedioxy-2-propyltoluene (referred to as piperonyl butoxide), 1,2-methylenedioxy-4-(2-octylsulfinyl)propyl)benzene (referred to as sulfoxide), 4-(3,4-methylenedioxyphenyl)-5-methyl-1,3-dioxane (referred to as Safroxan), N-(2-ethylhexyl)-bicyclo[2,2,1]hepta-5-en-2,3-dicarboxyimide (referred to as MGK-264), octachlorodipropylether (referred to as S-421), isobornylthiocyanoacetate (referred to as Thanite), etc., and other known synergists effective for allethrin or pyrethrin to increase the insecticidal effect of the compounds.

The compounds of this invention, unlike the ordinary chrysanthemumic acid ester type compounds, are relatively stable to light, heat and oxidation, but where particularly high stability is required in use under a very severe oxidative condition, it is recommended to add a suitable quantity of an antioxidant or an ultraviolet absorber, for example, a phenol derivative such as BHT or BHA, a bis-phenol derivative, an arylamine such as phenyl-α-naphthylamine, phenyl-β-naphthylamine, a condensate of phenetidine and acetone etc., or a benzophenone type compound to obtain a effectively stable composition.

It is also possible to obtain a multiple-purpose composition with excellent effect by mixing other physiologically active substance such as, for example, allethrin, N-(chrysanthemoxymethyl)-3,4,5,6-tetrahydrophthalimide (referred to as tetramethrin), 5-benzyl-3-furylmethyl chrysanthemate (referred to as resmethrin), 3-phenoxybenzyl chrysanthemate,

5-propargylfurfuryl chrysanthemate, 2-methyl-5-propargyl-3-furylmethyl chrysanthemate and d-trans-chrysanthemum-monocarboxylic acid esters, d-cis,trans-chrysanthemum-monocarboxylic acid esters thereof, or pyrethrum extract, d-trans-chrysanthemum-monocarboxylic or d-cis,trans-chrysanthemum-monocarboxylic esters of d-allethrolone, and other known cyclopropanecarboxylic acid esters, or an organic phosphorus type insecticide such as O,O-dimethyl O-(3-methyl-4-nitrophenyl) phosphorothioate (referred to as fenitrothion), O,O-dimethyl O-4-cyanophenyl phosphorothioate (hereinafter referred to as cyanofos), O,O-dimethyl O-(2,2-dichlorovinyl) phosphate (referred to as dichlorvos) or the like, a carbamate type insecticide such as 1-naphthyl N-methyl carbamate, 3,4-dimethylphenyl N-methyl carbamate, m-tolyl N-methyl carbamate, O-sec-butylphenyl N-methyl carbamate, O-iso-propoxyphenyl N-methyl carbamate or the like, and other insecticides or fungicides, nematocide, acaricide, herbicide, plant growth regulator, fertilizer, microbial agricultural chemicals such as BT or BM preparation, insect hormone preparation and other agricultural chemicals. A high synergistic effect may be derived from blending of these chemicals.

In the preparation of an insecticidal and/or acaricidal composition, the content of the compound (I) may be from 0.1 to 95% by weight. Practical embodiments of the insecticidal and/or acaricidal composition

according to the invention are illustratively shown in the following examples wherein parts are by weight.

Shown below are preparation examples of insecticides and/or acaricides using the compounds of this invention.

Preparation Example 1

To 10 parts of each of the compounds (1) to (9) of this invention are added 15 parts of an emulsifier (Sorpol 3005-X, registered trademark of Toho Chemicals) and 75 parts of xylene, and each mixture is well stirred for mixing and dissolving the materials to obtain an emulsifiable concentrate.

Preparation Example 2

To 1 part of each of the compounds (1) to (9) of this invention is added 0.3 part of PAP (isopropylacidophosphate), and each mixture is dissolved in 20 parts of acetone, further added with 98.7 parts of 300-mesh clay and then sufficiently stirred and mixed in a mortar, and thereafter acetone is evaporated away to obtain a dust.

Preparation Example 3

To 0.2 part of each of the compounds (1) to (9) of this invention are added 2 parts of m-tolyl-N-methylcarbamate and 0.3 part of PAP (above-mentioned), and each mixture is dissolved in 20 parts

of acetone, added with 97.5 parts of 300-mesh clay and then sufficiently stirred and mixed in a mortar, followed by removal of acetone by evaporation to obtain a dust.

Preparation Example 4

To 10 parts of each of the compounds (1) to (9) of this invention is well mixed 5 parts of an emulsifier (Sorpol SM-200, registered trademark of Toho Chemicals), and each mixture is further added with 85 parts of 300-mesh diatomaceous earth and then sufficiently stirred and mixed in a mortar to obtain a wettable powder.

Preparation Example 5

To 10 parts of each of the compounds (1), (3) and (9) of this invention is added 5 parts of 1-naphthyl-N-methylcarbamate, followed by mixing of 5 parts of an emulsifier (Sorpol SM-200 (above-mentioned)), and each mixture is further added with 80 parts of 300-mesh diatomaceous earth and sufficiently stirred and mixed in a mortar to obtain a wettable powder.

Preparation Example 6

0.5 Part of each of the compounds (1) to (9) of this invention is dissolved in deodorized kerosene in an amount that makes 100 parts in all, thereby obtaining an oil solution.

Preparation Example 7

0.4 Part of the compound (2) of this invention, 0.2 part of tetramethrin (above-mentioned), 7 parts of xylene and 7.4 parts of deodorized kerosene are mixed and dissolved, and the mixture is charged into an aerosol container. After attaching a valve portion to the container, 85 parts of a propellant (liquefied petroleum gas) is charged under pressure through said valve portion to obtain an aerosol.

Preparation Example 8

0.4 Part of the compound (1) of this invention, 0.2 part of resmethrin, 13.4 parts of deodorized kerosene and 1 part of an emulsifier (Atmos 300, a registered trademark of Atlas Chemicals) are mixed, and the mixture is added with water and emulsified and then charged into an aerosol container together with 35 parts of a 3:1 mixture of deodorized butane and deodorized propane to obtain a water based aerosol.

Preparation Example 9

0.3 Gram of allethrin is added to 0.3 g of the compound (1) of this invention, and the mixture is dissolved in 20 ml of methanol and further uniformly mixed with 99.4 g of a carrier (a 3:5:1 mixture of Tabu powder/marc/wood flour). After evaporating away methanol, the residual mixture is added with 150 ml of water, sufficiently kneaded, and then molded and dried

to obtain a mosquito coil.

Preparation Example 10

To 0.1 g of the compound (1) of this invention is added 0.02 g of d-trans-chrysanthemum-carboxylic acid ester, 0.05 g of BHT and 0.1 g of piperonyl butoxide, and the mixture is dissolved in a suitable quantity of chloroform and uniformly adsorbed on the surface of a 3.5 cm x 1.5 cm x 0.3 cm (thickness) asbestos, and a piece of asbestos of the same size is pasted to said surface, thereby obtaining a fiber fumigant which, in use, is heated on an electric heating plate. A pulp plate or the like capable of producing the same effect may be used instead of asbestos as the fibrous carrier.

Preparation Example 11

To 80 parts of each of the compounds (2) and (5) of this invention are added 10 parts of an emulsifier (Sorpol 3005-X, registered trademark of Toho Chemicals) and 10 parts of xylene, and each mixture is well stirred for mixing and dissolving the materials to obtain an emulsifiable concentrate.

The thus obtained compositions of this invention have the below-described insecticidal effect.

Test Example 1 Killing effect against houseflies

At the bottom of a polyethylene cup with a diameter of 5.5 cm was placed a filter paper of the same

size, and then 0.7 ml of a 200 times water-diluted liquid (corresponding to 500 ppm) of the emulsifiable concentrate obtained in Preparation Example 1 was added dropwise onto the filter paper. 30 Milligrams of sucrose was placed on the filter paper as bait. Then 10 female housefly adults were released in the cup and the cup was covered. 48 Hours thereafter, the dead and alive were observed to determine the mortality. (The test was repeated twice).

| Compounds tested | Mortality (%) |
|---|---|
| (1) | 100 |
| (2) | 100 |
| (4) | 100 |
| (5) | 100 |
| (6) | 100 |
| (7) | 100 |
| (8) | 100 |
| (9) | 100 |
| No treatment | 0 |

Test Example 2 Knockdown effect on German cockroach

Each of the compounds (1) to (7) of this invention and common known pyrethroids were dissolved in deodorized kerosene to give oil solutions as shown below. Ten German cockroach adults were released in a plastic cup and covered with 50 mesh nylon gauze.

0.6 Ml of the oil solution was sprayed to cockroaches from the 50 cm height, and the number of knockdowned insects was counted and a $KT_{50}$ value was calculated.

| Compound tested | Concentration of oil solution and $KT_{50}$ (min.) | | |
|---|---|---|---|
| | 0.025% | 0.0125% | 0.00625% |
| (1) | 1.2 | 2.3 | 3.9 |
| (2) | 4.1 | 4.6 | 5.5 |
| (3) | 3.8 | 4.4 | 5.6 |
| (4) | 4.3 | 5.8 | 8.6 |
| (5) | 4.5 | 6.0 | 10.5 |
| (6) | 3.5 | 5.2 | 6.0 |
| (7) | 3.3 | 4.0 | 5.8 |
| Fenpropathrin | 8.3 | 14.9 | >20 |
| Fenvalerate | 14.7 | >20 | - |
| A* | 4.6 | 6.5 | 19.2 |
| Permethrin | >20 | - | - |
| Cypermethrin | 20.0 | >20 | - |

* A: 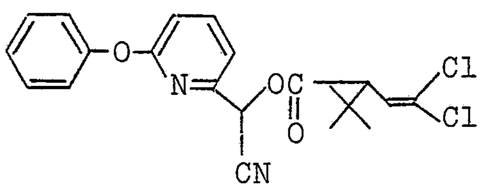 (Published Unexamined Japanese Patent Application No. 112881/1978)

The compounds of this invention showed extremely high knockdown activity in comparison with related pyrethroid compounds as shown in this Table.

Test Example 3 Killing effect on Spodoptera litura

The leaves of cabbage were immersed in a diluted solution of the emulsifiable concentrate of Preparation Example 1 (diluted with water to a predetermined concentration) for one minute and then air-dried. The thus treated leaves and 10 fourth-instar larvae of Spondoptera litura were placed in the plastic cups with a diameter of 9 cm and a height of 4 cm. The cups were placed in a room of a 26°C artificial atmosphere and the dead and alive were examined to determined the mortality 24 hours thereafter.

| Compounds tested | Concentration (ppm) and mortality (%) | |
|---|---|---|
| | 500 | 100 |
| (1) | 100 | 100 |
| (2) | 100 | 100 |
| (3) | 100 | 100 |
| (4) | 100 | 100 |
| (5) | 100 | 100 |
| (9) | 100 | 100 |

Test Example 4 Controlling effect against Tetranychus cinnabarinus

Four leaves of potted kidney bean plant of 5 days after sowing, were parasitized with the adult female Tetranychus cinnabarinus at a rate of 10 insects

per leaf and kept in a 27°C constant temperature room.

6 Days after, a solution prepared by diluting the emulsifiable concentrate of Preparation Example 1 with water to a 500 ppm active ingredient concentration was sprayed at a rate of 10 cc per pot on a turntable. 10 Days thereafter, the number of the insects parasitic on the plant was counted. Judgment of the controlling effect of the compounds was based on the following rating:

++ 0-9 insects are parasitic per leaf

\+ 10-30 insects are parasitic per leaf

\- more than 30 insects are parasitic per leaf

The results are shown in the following table.

| Compounds of this invention | Judgment |
|---|---|
| (1) | ++ |
| (2) | ++ |
| Chlorodimeform* | ++ |
| No treatment | - |

* 1,000 times diluted solution of a 50% emulsion of N'-(2-methyl-4-chlorophenyl)-N,N-dimethylformamide

Test Example 5

The dust obtained in Preparation Example 3 was dusted by Bell jar duster to the tillering-stage rice plants in the 1/10,000-are Wagner pots at a rate of 3 kg/10 ares, and the plants were covered with the wire net cages. Then about 15 each of adult Nephotettix cincticeps and Laodelphax striatellus were released and fed in each cage. The pots were kept in a greenhouse and the dead and alive of the insects were observed 24 hours after release of the insects. Each of said powder was capable of killing said both species of adult 100%.

Test Example 6

5 Ml each of the oil solution obtained in Preparation Example 6 was sprayed to the housefly adults (about 100 flies per group) according to the Campbell's turn-table method (Soap and Sanitary chemicals, Vol. 14, No. 6, p. 119 (1938)), and when the flies were exposed to descending mist of the oil preparation for 10 minutes, the flies were killed 100% by any of the oil solution.

Test Example 7

The adult housefly-killing effect of the aerosols obtained in Preparation Examples 7 and 8 was examined according to an aerosol test method (CSMA method) using a Peet Grady chamber (6 $ft^3$). The results

showed that either of the aerosols could knock down 80% or more of the flies at 15 minutes after spray, and 90% or more of the flies were dead at 24 hours after spray.

WHAT IS CLAIMED IS:

1. Carboxylates of the formula (I),

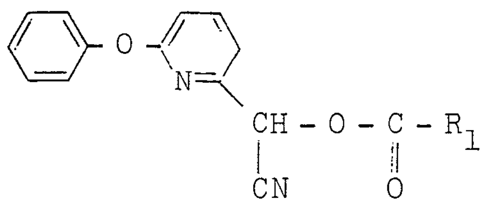

(I)

wherein $R_1$ is

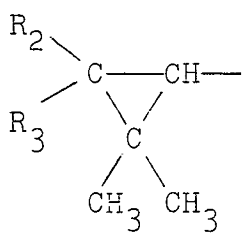

(II) or

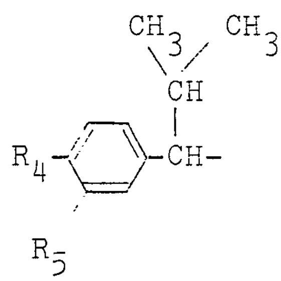

(III)

wherein $R_2$ is a hydrogen atom or a methyl group; $R_3$ is a 2-methyl-1-propenyl group when $R_2$ is a hydrogen atom, and $R_3$ is a methyl group when $R_2$ is a methyl group; each of $R_4$ and $R_5$ is a hydrogen atom, a fluorine, chlorine, or bromine atom, or a methyl, methoxy or 3,4-methlenedioxy group, with the proviso that $R_4$ and $R_5$ are not hydrogen atoms at the same time.

2. The compound according to Claim 1, wherein $R_1$ is

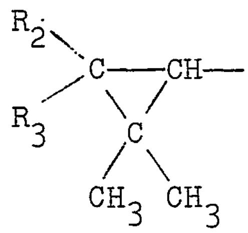

(II)

wherein $R_2$ and $R_3$ are as defined in Claim 1.

3. The compound according to Claim 1, wherein $R_1$ is

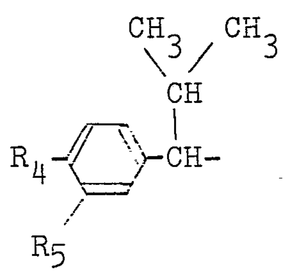

(III)

wherein $R_3$ and $R_4$ are as defined in Claim 1.

4. 6-Phenoxy-2-pyridyl-α-cyanomethyl 2,2,3,3-tetramethylcyclopropanecarboxylate.

5. 6-Phenoxy-2-pyridyl-α-cyanomethyl d,l-cis,trans-chrysanthemate.

6. 6-Phenoxy-2-pyridyl-α-cyanomethyl 2-(4-chlorophenyl)isovalerate.

7. 6-Phenoxy-2-pyridyl-α-cyanomethyl 2-(4-methoxyphenyl)isovalerate.

8. 6-Phenoxy-2-pyridyl-α-cyanomethyl 2-(3,4-methylenedioxyphenyl)isovalerate.

9. A process for producing the compounds according to Claim 1, which comprises reacting either

a) a carboxylic acid of the formula (IV):

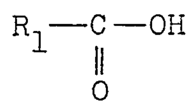

(IV)

wherein $R_1$ is as defined in Claim 1, or a reactive derivative thereof with an alcohol or its reactive derivative of the formula (V):

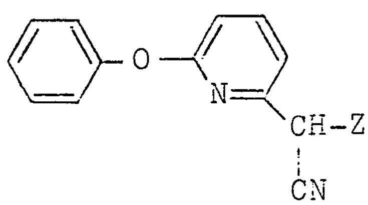

(V)

wherein Z is a hydroxy group or a chlorine or bromine atom, or

b) a carboxylic acid halide of the formula (VI):

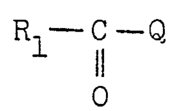

(VI)

wherein Q is a chlorine or bromine atom, and $R_1$ is as defined in Claim 1, with an alcohol of the formula (VII):

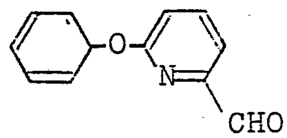

(VII)

and an alkali metal cyanide.

10. An insecticidal and/or acaricidal composition which comprises as an active ingredient an insecticidally and/or acaricidally effective amount of a compound according to Claim 1 and an inert carrier.

11. The use of the compounds according to Claim 1 as an insecticide and/or acaricide.